Europäisches Patentamt

European Patent Office

Office européen des brevets

(11) Publication number: **0 337 938**
**A2**

## (12) EUROPEAN PATENT APPLICATION

(21) Application number: **89810197.7**

(22) Date of filing: **14.03.89**

(51) Int. Cl.⁴: **A 61 K 31/565**
**A 61 K 31/57**

(30) Priority: **14.03.88 GB 8806028**
**10.06.88 GB 8813740**

(43) Date of publication of application:
**18.10.89 Bulletin 89/42**

(84) Designated Contracting States: **ES GR LU**

(71) Applicant: **SANDOZ AG**
**Lichtstrasse 35**
**CH-4002 Basel (CH)**

(72) Inventor: **Christiansen, Claus**
**Bakken 3**
**DK-2600 Glostrup (DK)**

**Riis, Bente Juel**
**Bakken 3**
**DK-2600 Glostrup (DK)**

The title of the invention has been amended (Guidelines for Examination in the EPO, A-III, 7.3).

(54) **19-Norprogesterone derivatives in the treatment of osteoporosis.**

㊉ The invention provides the use of a progestin unsubstituted in the 19 position in the manufacture of a medicament for the treatment of established osteoporosis on a non-cyclical basis.

EP 0 337 938 A2

Bundesdruckerei Berlin

**Description**

## OSTEOPOROSIS TREATMENT

This invention relates to the treatment of established osteoporosis.

Introduction

Osteoporosis is a disorder characterized by reduced amounts of bone mineral content (BMC or bone mass) per unit volume of bone. This is due to loss in mineral content of the bone, primarily calcium phosphate or other calcium salts, which is responsible for the density and hardness of the bone. This loss occurs as a result of complex as yet unexplained pharmacological interactions, one of which is an imbalance between the rate of bone formation and the rate of bone resorption (bone loss).

Bone is lost from all parts of the skeleton, including cortical bone which forms the bulk of the bone skeleton and predominates in long bones including the fingers, and trabecular bone which predominates in bone near joints e.g. in the spinal vertebrae and in the distal forearm.

Osteoporosis results in an increased susceptibility to bone fractures, particularly hip fractures, fractures of the distal radius bone (Colles' fractures) and especially vertebral fractures. Thus the clinical manifestations of osteoporosis include fractures and their complications. Fracture treatment, especially hip fracture, requires extensive hospitalization. Such fractures are associated with an increased risk of mortality. Vertebral fractures may however not be immediately recognized. They lead in time to spinal deformities, e.g. end plate deformities, wedge deformities, and compression deformities, resulting in painful hunched backs and irreversible loss in height.

Osteoporosis is a major public health problem, affecting as many as 1 out of 2 women in a 70 year old + population.

Our understanding in the area of osteoporosis is hampered by the lack of meaningful clinical trials. Bone formation rate can be followed by biochemical tests, e.g. plasma bone Gla protein (pBGP also known as osteocalcin) and serum alkaline phosphatase; bone turnover rate may be followed by retention of $^{99m}$ Technetium diphosphonate; bone resorption may be followed by fasting urinary calcium/creatinine ratios, fasting urinary hydroxyproline and serum phosphate.

The changes in bone mineral content itself are quite small and can only be reliably followed over an extended period of time, e.g. a year, and in trials having well matched groups of treated and non-treated (placebo) subjects. Sophisticated bone measurement techniques, e.g. as described by L.Nilas, J.Nucl.Med., 1987, 28, 960, have to be used with adequate controls throughout the trial period. The data has to be analysed carefully taking due account of the precision of the method employed in order to obtain meaningful quantitative results.

Measurement techniques used include single photon absorptiometry (using a I $^{125}$ source with photopeak at 27 KeV for bone near the joint of the ulna and radius bones in the distal forearm ($BMC_{prox}$ and $BMC_{dist}$) and dual photon absorptiometry (using a gadolinium - $^{153}$ source with photopeaks at 44 and 100 KeV for the vertebrae ($BMC_{spine}$) and for the total body bone mineral (TBBM).

The BMC suddenly starts to decrease within a year of the menopause, oophorectomy (removal of the ovaries) or other dysfunction of the ovaries. The loss is initially rapid. Forearm bone loss is of the order of 3 to 4 per cent per year and the spinal bone loss even higher. Over a ten year period 20 to 30 per cent BMC will be lost.

After a menopausal age of ten years the decrease in BMC at least in the forearm slows down. By this time however the BMC has passed through a so-called fracture threshold (corresponding to about 20 per cent loss in BMC). The bones have then sufficiently wasted away to become liable to fractures in e.g. the vertabrae.

A considerable amount of research in osteoporosis has concentrated on the prevention of postmenopausal osteoporosis, i.e. before or during the period when BMC is decreasing rapidly. Such therapy is directed against preventing or slowing down further loss in bone mass, rather than replacing bone already lost.

Estrogen therapy may be employed. This may be given not only to prevent osteoporosis, but also to treat climacteric symptoms associated with the menopause.

Up till now despite the great need there is no effective, safe, well tolerated, orally administrable treatment of established osteoporosis, e.g. manifest senile or postmenopausal osteoporosis. Such a therapy ideally significantly increases bone mass to above the fracture threshold in a completely different class of patients i.e. much older post-menopausal subjects who have already experienced material loss in BMC, and accordingly have too low a bone mass. This may alternatively or additionally be evidenced by at least one fracture.

We have now found that administration of a progestin unsubstituted in the 19 position and especially as NETA (norethisterone acetate) on a continuous basis, i.e. for longer than 10 days a month, and optionally in the presence of an estrogen leads to a clear and unexpected increase in trabecular and cortical BMC. Moreover, we have found that such therapy is especially and unexpectedly well tolerated, e.g. by the lack of inconvenient bleedings and by satisfactory gynaecological examinations.

There has understandably been a reluctance to administer progestins and estrogens to senile women on a long term continuous basis. Thus progestin/estrogen treatment of climacteric symptoms is usually stopped after a few years. This was mostly on the basis that undesired vaginal bleedings or other side effects may be caused by the broad pharmacological actions of the progestins and estrogens.

Unlike some previous trials using old post-menopausal women with spinal crush fractures and thus a severe degree of osteoporosis, in the trial forming the basis of the present invention, the participants selected had osteoporosis of a moderate degree with a bone mineral content on the average 25% below premenopausal women. This permitted the setting up of two well-matched groups.

The use of a progestin unsubstituted in the 19 position such as NETA in the method of the invention is indicated by a significant increase in bone mineral content and decrease in bone resorption and formation, as measured in the following clinical trial.

In this trial, calcium and bone metabolism is examined in a group of women with a moderate degree of osteoporosis who received, on a continuous basis, either a combination of 2 mg 17-beta-oestradiol and 1 mg of norethisterone acetate (hereinafter these patients are referred to as the hormone-treated group) or placebo (hereinafter referred to as the placebo group).

Patients and Methods

Subjects

This study was double-blind clinical controlled trial. All participants were informed verbally and in writing about the trial, and all gave their informed consent, in accordance with Helsinki Declaration II. The participants were selected by the following procedures:-

1. 4900 spinal x-rays of women age 55 to 75 years taken during a 5 year period at a hospital were assessed. 219 women were found to have at least one vertebral fracture (wedge or compression). Of these, 81 were excluded because of malignant, gastrointestinal, metabolic, or rheumatic disease, or drug intake known to affect calcium metabolism. Earlier transitory use of hormonal substitution was not an exclusion criterion of the remaining 138 patients, 44 consented to enter the study and to receive an experimental therapy of osteoporosis. After the introductory investigation 5 patients dropped out.

2. 316 patients referred because of distal forearm fracture during the preceeding 5 years were approached and the same criteria were applied. Of 316 women 133 were excluded. 80 of the remaining 183 agreed to participate. From these two populations 20 women with vertebral fractures and 20 women with distal forearm fractures were randomly selected for the present study.

The women were allocated by random numbers to two treatment groups, blindly receiving either continuous hormone therapy of 2 mg 17-beta-oestradiol + 1 mg norethisterone acetate in a tablet plus a calcium supplementation of 500 mg Calcium (Calcium Sandoz $^R$) in the form of an effervescent tablet containing 0.3 g calcium carbonate and 2.94 g calcium lactate gluconate (n = 20; 10 with spinal fractures, 10 with Colles' fractures) or a placebo tablet combined with the same calcium supplementation (n = 20; 10 with spinal fractures, 10 with Colles' fractures). The treatment lasted for one year, during which all patients were examined every three months (five examinations in all). Seven patients in the hormone group continued with the treatment for a further year and were studied at the end of the second year.

Compliance to trial

Of the 40 women who entered the trial, 31 (78%) completed the one year of treatment. In the hormone group 4 women dropped out (2 with Colles' fracture, 2 with spinal fracture). One woman was killed in a car accident, one woman dropped out because she started treatment with digitalis due to incompensate heart disease, one woman dropped out due to unacceptable breast-tenderness, and one due to a tumor discovered in the breast (benign). In the placebo group 5 women dropped out (2 with Colles' fractures, 3 with spinal fractures). One woman died from unrecorded reasons, one woman dropped out due to aggravation of heart disease, one due to general indisposition of unrecorded reason, one due to aggravation of eczema, and one dropped out due to personal reasons. This left 16 women in the hormone group and 15 women in the placebo group.

METHOD

Bone mass measurements

The principles of measurement have been reported in J.Nucl.Med., 1987, 28, 960:

Bone mineral content (BMC) of the forearms was measured by single photon absorptiometry, using $^{125}$I. The method estimates BMC in a proximal (BMC $_{prox}$) and distal (BMC $_{dist}$) region of the forearm with a trabecular bone content of 13% and 55% respectively (Eggstein M, Kreutz FH. Eine neue Bestimmung der Neutralfette im Blutserum und Gewebe. Klin.Wochenschrift 1966; 44; 262-273). The values are expressed as units corresponding to grams of mineral per cm of bone length. The long-term in vivo precision of proximal and distal BMC is 1% and 1.5% (Nilas L., Borg J, Gotfredsen A, Christiansen C. -Comparison of single- and dual-photon absorptiometry in postmenopausal bone mineral loss. J.Nucl.Med.1985; 26; 1257- 1262). In case of forearm fracture we measured the non-fractured forearm. In case of bilateral fractures occurring at different times we measured the arm with the "oldest" fracture. In case of bilateral fractures occurred at the same time we measured both forearms.

Bone mineral content of the lumbar spine (BMC$_{spine}$) was measured by dual photon absorptiometry on a Lunar Radiation Corporation DP3 scanner. BMC$_{spine}$ was calculated as the BMC values obtained in vertebrae L2, L3, and L4. The long-term in vivo precision is 4%, after correcting the values for the errors due to source chance (Nilas L., Borg J, Gotfredsen A, Christiansen C. - Comparison of single- and dual-photon absorptiometry in postmenopausal bone mineral loss. J.Nucl.Med.1985; 26; 1257-1262).

Bone mineral content of the total skeleton (TBBM) was likewise measured by dual photon absorptiometry on a whole body scanner developed in our laboratory. The long-term in vivo precision of TBBM is about 2.5% (Gotfredsen A, Borg J, Christiansen C, Mazess RB. Total body bone mineral in vivo by dual photon absorptiometry. I. Measurement procedures. Clin.Physiol.1984; 4; 343-355), after correcting for source chance.

Blood samples were taken and urine collected in the morning after an overnight fast and tobacco abstinence.

Biochemical estimates of calcium metabolism

Serum alkaline phosphatase (sAP) was measured enzymatically according to Scandinavian recommendation (The Committee on Enzymes of the Scandinavian Society for Clinical Chemistry and Clinical Physiology. Recommended methods for the determination of four enzymes in blood. Scand.J.Clin.Lab.Invest.1974; 33; 291-306). Serum calcium was determined by atomic absorptiometry and corrected to a plasma protein concentration of 70 q/l. Serum phosphate (s-Phosphate) was measured by spectrophotometry. Plasma bone Gla protein (pBGP) was measured by radioimmunoassays (Johansen JS, Hansen JEM, Christiansen C. A radioimmunoassay for bone Gla protein (pBGP) in human plasma. Acta Endocrinol 1987; 114; 410-416). Fasting urinary calcium and creatinine were measured on a SMA 6/6 autoanalyser, and the calcium excretion was corrected for creatinine excretion (FU Ca/Cr). Fasting urinary hydroxyproline was measured by a spectrophotometric method (Podenphant J, Larsen N-E, Christiansen C. An easy and reliable method for determination of urinary hydroxyproline. Clin.Chim.Acta 1984; 142; 145-148), and corrected for creatinine excretion (FU Hpr/Cr).

Radiopharmaceutical measurement of bone turnover

Whole body retention (WBR) of $^{99m}$Technetium-diphosphonate ($^{99m}$Tc-DP) was measured using a widefield gamma camera with a diverging parallel (fishtail) collimator. 2 mCi of $^{99m}$Tc-DP was injected intravenously and the whole body measurement was started 5 min. later. Scanning was repeated at 24 hours and WBR was calculated after subtracting background radiation and correcting for radioactive decay by using the mean count at 24 hours. WBR is expressed as per cent of the retained radioactivity.

The method has a precision of 4.8% (Thomsen K, Nilas L, Mogensen T, Christiansen C. Determination of bone turnover by urinary excretion of $^{99m}$Tc-MDP. Eur.J.Nucl.Med. 1986; 12, 342-345).

Serum lipids and lipoproteins

The HDL lipoprotein fraction was separated from chylomicrons, LDL, and very low-density lipoprotein, (VLDL) by the $Mg^{2+}$/dextran sulphate precipitation technique (Finley RP, Schifman RB, Williams RJ, Lichti DA. Cholesterol in high-density lipoprotein:Use of $Mg^{2+}$/Dextran sulfate in its enzymic measurement. Clin.Chem.1978; 24, 931-933). Total serum cholesterol and HDL cholesterol were by an enzymatic technique with use of an LKB Ultrolab system. The intra-assay and interassay variations were 1% and 2% (total cholesterol) and 2% and 5% (HDL cholsterol), respectively (Allain CC, Poon LS, Chan SCG, Richmond W, Fu PC. Enzymatic determination of total serum cholesterol. Clin.Chem.1974; 20, 470-475). Serum triglycerides were determined by an enzymatic technique with use of an LKB 8600 reaction rate analyzer. The intra-assay and inter-assay variations of this method are 5% and 8%. Serum LDL cholesterol was then estimated according to the procedure of Friedewald et al. (Friedewald WT, Levy RI, Frederickson DS. Estimation of the concentration of low-density lipoprotein cholesterol in plasma, without use of the preparative ultracentrifuge. Clin.Chem. 1972; 18, 499-502). The intra-assay and inter-assay variations of this estimation were 5% and 10%.

Uterine bleedings

Bleedings were recorded on a day-to-day questionnaire delivered at each examination.

Safety parameters

Serum aspartate aminotransferase (s-ASAT), serum creatinine (s-Crea), haemoglobin (hgb), and blood pressure (BP) were measured by routine procedures.

Statistical analysis

Clinical data and initial values of the measured variables were compared by Student's t test for unpaired data (table I). All initial values were set at 100%, and subsequent values were expressed in per cent of initial values. Differences within groups were evaluated by Student's t test for paired data and between groups by Student's t test for unpaired data. *corresponds to $p < 0.05$; xx to $p < 0.01$ and xxx $p = < 0.005$, except where otherwise defined.

RESULTS

Because the response to treatment was not significantly different in the group with Colles' fractures and the group with vertebral fractures the groups were pooled.

Table I gives the clinical data and initial values of bone mineral measurements. By chance, the hormone groups had approximately 5 kg less that the placebo grouup however, the difference was not statistically significant. The two groups were otherwise well matched.

Table I

CLINICAL DATA (Mean ± 1SD)

| | HOR-MONE GROUP | SIGNIFI-CANCE OF DIF-FERENCE | PLACEBO GROUP |
|---|---|---|---|
| Age (years) | 63.6 ± 5.0 | NS | 64.8 ± 5.7 |
| Menopausal age (years) | 16 ± 6 | NS | 18 ± 7 |
| Height (cm) | 159.7 ± 9.4 | NS | 159.3 ± 6.1 |
| Weight (kg) | 59.9 ± 8.3 | NS | 65.2 ± 9.9 |
| $BMC_{prox}$ (units) | 27.4 ± 5.1 | NS | 27.4 ± 6.2 |
| $BMC_{dist}$ (units) | 24.7 ± 4.3 | NS | 26.6 ± 5.6 |
| $BMC_{spine}$ (g) | 35.4 ± 8.4 | NS | 33.4 ± 6.4 |
| TBBM (g) | 2534 ± 558 | NS | 2586 ± 493 |

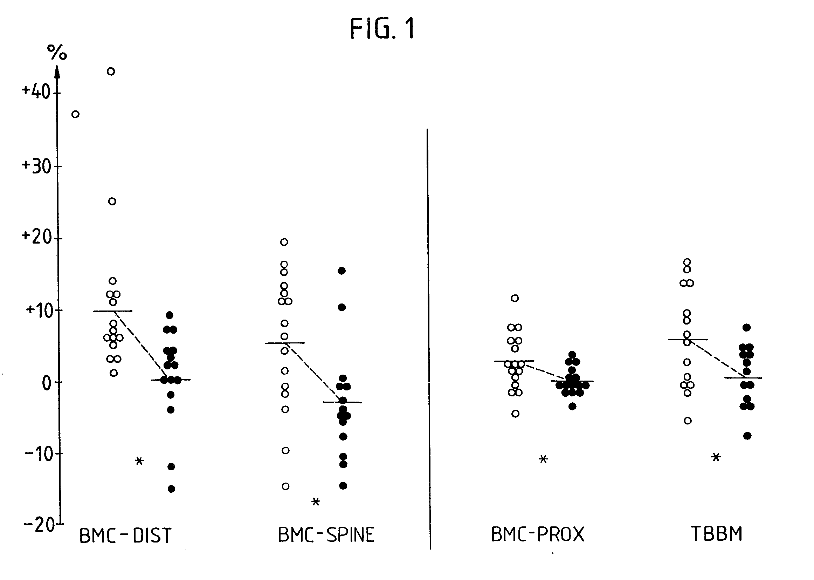

Accompanying Figure 1 shows the individual changes (in percent change) positive or negative in bone mineral content during the year of treatment. The hormone group data are shown as unfilled circles, the placebo group as filled circles. BMC dist and BMC spine relate mainly to trabecular bone, BMC prox and TBBM mainly to cortical bone.

At all four measurement sites the mean bone mineral content was greater in the hormone group as compared to the placebo group ($p < 0.05$). In $BMC_{dist}$ and $BMC_{spine}$ there was a difference between the hormone and the placebo groups of approximately 8%, whereas in $BMC_{prox}$ and TBBM the differences were approximately 3% and 5%, respectively.

Compared to baseline values the placebo group did not change significantly in $BMC_{dist}$, $BMC_{prox}$ or TBBM. Also the decrease in $BMC_{spine}$ of 3.2% was not statistically significantly different from baseline values.

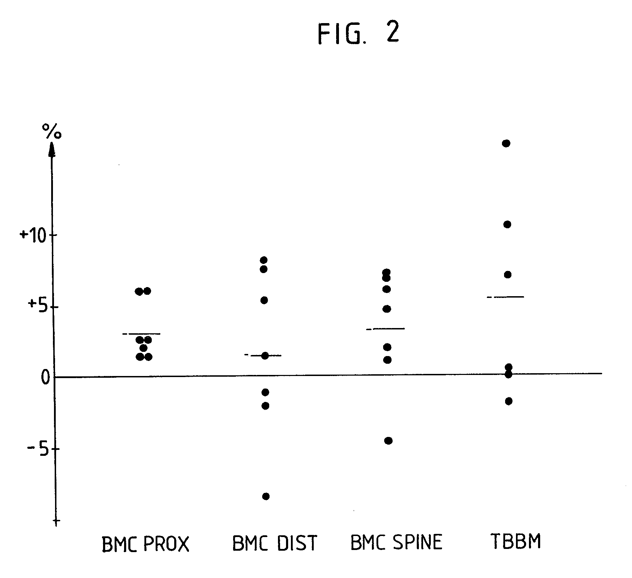

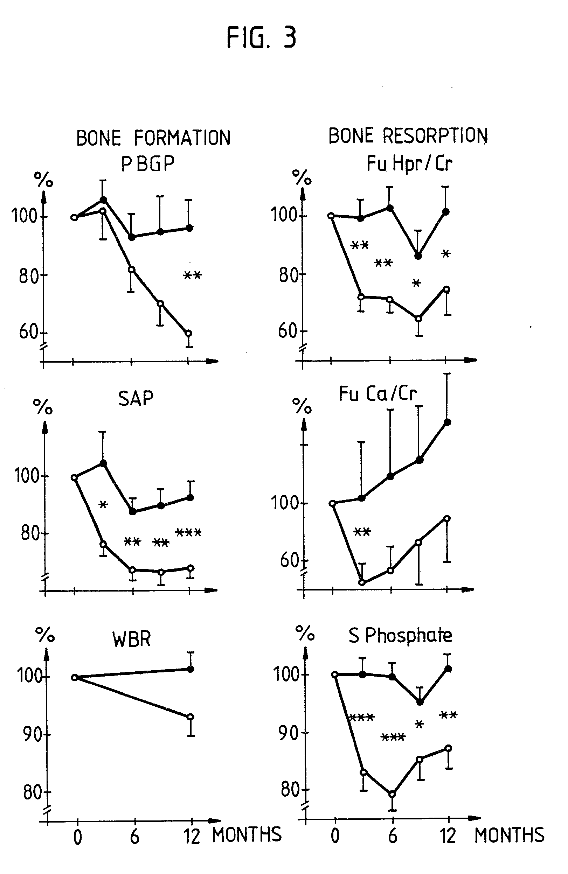

Accompanying Figure 2 shows individual changes (filled circles) in bone mass measurements during the second year of treatment (i.e. starting after one year of treatment) for women treated with the hormones. BMC prox, BMC dist, BMC spine and TBBM are shown. The women had on average an increased bone mass of 1.5 to 5.5 per cent and out of 28 changes only 5 were below zero. These results indicate that the steady state had not been achieved even within 2 years.

Accompanying Figure 2 shows the changes in the biochemical estimates after 3,6,9 and 12 months of treatment indicating that decreases in bone formation and bone resorption had taken place in the hormone treated group (unfilled circles) compared to the placebo group (filled circles). The values are shown as percent of the initial values. In the hormone treated group there were significant decreases in pBGP, sAP, FU Hpr/Cr, FU Ca/Cr and s-phosphate when compared to the placebo group. WBR (taken only at the end of 1 year) also decreased in the hormone treated group as compared to the placebo group, but the decrease was just of borderline significance (t = 1.9).

During the second year the mean values remained virtually unchanged. From a comparison of biochemical data on the hormone group in this trial with that of a group of early postmenopausal women receiving unopposed estrogen treatment in another trial, it appears that the elderly women decrease more in bone resorption parameters, and the early postmenopausal women decrease more in bone formation parameters.

Following Table II shows the initial values at the beginning of the first year and the final values at the end of the trials expressed as a percentage of the initial values, of serum lipids and lipoproteins. The initial values were similar in the two groups. No significant change was observed in the placebo group. The changes in the hormone group showed a significant decrease in serum triglycerides ($p < 0.01$), serum total cholesterol ($p < 0.001$), serum LDL cholesterol ($p < 0.001$) and serum HDL cholesterol ($p < 0.05$). The overall effect was beneficial. HDL cholesterol levels only decreased by a small amount. Also LDL cholesterol levels decreased. All values renamed virtually stable during the second year of treatment.

Table II

SERUM LIPIDS AND LIPOPROTEINS BEFORE AND AFTER ONE YEAR OF TREATMENT WITH HORMONES OR PLACEBO (MEAN ± 1SD).

| | n = 16 HORMONES | | n = 15 PLACEBO | |
|---|---|---|---|---|
| | Initial | % Change | Initial | % Change |
| S-Triglycerides mmol/l | 1.16 ± 0.38 | 88.0 ± 14.7 ** | 1.36 ± 0.75 | 103.7 ± 27.1 |
| Total S-cholesterol mmol/l | 6.82 ± 1.30 | 88.0 ± 9.0 *** | 7.86 ± 2.16 | 99.3 ± 9.6 |
| S-LDL | 4.87 ± 1.25 | 88.9 ± 11.3 *** | 5.70 ± 2.12 | 96.7 ± 11.3 |
| S-HDL | 1.45 ± 0.27 | 92.2 ± 14.3 * | 1.55 ± 0.47 | 110.7 ± 13.0 |

* = $p < 0.005$;
** = $p < 0.01$;
*** = $p < 0.001$ (paired t test)

Following Table III shows the initial values at the beginning of the first year and the final values at the end of the first year (expressed as per cent of the initial values) in the measured safety parameters. The hormone group had a mean systolic blood pressure which was significantly lower than that of the placebo group ($p < 0.05$). There were no significant changes in the two groups in any of the variables, except for serum calcium ($p < 0.001$) and diastolic blood pressure ($p < 0.05$) which decreased significantly in the hormone group. Six women in the hormone group experienced uterine bleedings during the treatment. In all cases the bleedings were small, and would more precisely be designated a brown flour vaginalis. In 4 women this event took place only within the first three treatment months, one woman had a bleeding in the fourth treatment months and one woman had small bleedings in the third, fourth and sixth treatment months. All gynaecological examinations and smear tests were normal.

In the placebo group one woman had 3 small bleedings during the last six treatment months due to a senile colpitis.

Table III

SAFETY PARAMETERS BEFORE AND AFTER ONE YEAR OF TREATMENT (MEAN $\pm$ 1SD).

| | HORMONES | | PLACEBO | |
| | Initial value | % Change | Initial value | % Change |
|---|---|---|---|---|
| S-ASAT | 22 ± 5 | 90.1 ± 18.7 | 24 ± 5 | 96.7 ± 15.1 |
| Hgb mmol/l | 9.1 ± 1.0 | 99.4 ± 4.6 | 8.6 ± 0.5 | 98.0 ± 3.5 |
| BP syst mm Hg | 138 ± 14 | 97.9 ± 10.7 * | 150 ± 14 | 98.1 ± 8.2 |
| BP dia mm Hg | 83 ± 8 | 93.3 ± 8.9 | 88 ± 7 | 99.5 ± 8.1 |
| Body weight (Kg) | 59.9 ± 8.3 | 100.9 ± 3.5 | 65.2 ± 9.9 | 100.8 ± 2.9 |
| Height (cm) | 159.7 ± 9.4 | 99.9 ± 0.4 | 159.3 ± 6.1 | 99.9 ± 0.4 |
| S-Crea mmol/l | 0.08 ± 0.01 | 101.7 ± 11.3 *** | 0.08 ± 0.01 | 98.9 ± 5.9 |
| S-Ca mmol/70 g | 2.41 ± 0.06 | 96.1 ± 3.7 | 2.43 ± 0.09 | 98.9 ± 3.9 |

* = p<0.05;
*** = p<0.001 (paired t test).

EP 0 337 938 A2

EP 0 337 938 A2

EVALUATION

The increase in bone mass induced by the method of the invention was more pronounced in the measured parts of the skeleton with a rather high content of trabecular bone (distal forearm, spine) than in parts of the skeleton with a high content of cortical bone (proximal forearm, total skeleton) (8% vs 3-5%), i.e. the method of the invention seems to be more active in the trabecular bone.

Thus if women had a bone mass measurement at a time where the bone loss was not too severe, e.g. when a forearm fracture occurred, 2, 3 or more years using method of the invention is indicated to induce an important increase in BMC even if the increase levels off after a couple of years. The resultant new bone is of normal structure and histology indicating an important decrease in fracture risk.

The hormone treatment significantly reduced the biochemical estimates of bone turnover into a premenopausal level, whereas these variables were virtually unchanged in the placebo group. The somewhat fluctuating response in FU Ca/Cr is probably a combination of the calcium supplement and some difficulties for senile women to undergo strict fasting.

The treatment regimen of the method of the invention did not produce significant side effects and was generally well accepted.

DESCRIPTION OF THE INVENTION

Accordingly the invention provides in one aspect:

a) the use of a progestin unsubstituted in the 19 position in the treatment of established osteoporosis, on a non-cyclical basis,

b) the use of a progestin unsubstituted in the 19 position in the manufacture of a medicament for the treatment of established osteoporosis on a non-cyclical basis, or

c) a method for the treatment of established osteoporosis in a subject which comprises administering a progestin unsubstituted in the 19 position to a subject in need of such treatent on a non-cyclical basis.

The invention provides in another aspect:-

a) the use of a progestin unsubstituted in the 19 position for increasing bone mass in a subject suffering from established osteoporosis, on a non-cyclical basis,

b) the use of a progestin unsubstituted in the 19 position in the manufacture of a medicament for increasing bone mass in the treatment of established osteoporosis on a non-cyclical basis, or

c) a method for increasing bone mass in a subject suffering from established osteoporosis which comprises administering a progestin unsubstituted in the 19 position to a subject in need of such treatment on a non-cyclical basis.

The present invention provides thus a dosage regimen which comprises administering a progestin unsubstituted in the 19 position (hereinafter a progestin of the invention) over a period of longer than 10 days, as compared to a cyclical regimen wherein the progestin is given for only part of the month and only estrogen is given for another part of the month.

The progestin is administered over preferably more than 15, 20, and especially more than 25 days, per month. Most conveniently the progestin is administered on a continuous basis, i.e. substantially every day, or, if in a sustained release form, every few days.

Progestins of the invention have no substituent (e.g. no methyl group) in the 19 position other than a hydrogen atom. They may have no substituent there at all if a double bond is present in ring A or B, and is linked to the 19 position.

Representative progestins of the invention include allylestrenol, demegestone, ethynodiol diacetate, gestonorone caproate, dl- and levo-noresterel, lynestrenol, methyl estrenolone (normethandrone), norethynodrel, norgestrienone, and quinestradiol or its acetate, and preferably norgestrel.

Particularly preferred examples of the progestin of the invention are norethisterone and derivatives thereof such as norethisterone carboxylic acid esters e.g. norethisterone enanthate and particularly norethisterone acetate (NETA).

The invention is applicable preferably to women over 50 years of age, especially to women over 60 years of age, more especially to women over 70 years of age. Conveniently the women are less than 85 years of age, e.g. less than 80 years of age.

Conveniently the subjects have experienced more than 15 per cent bone mass (or BMC or bone density) loss, especially more than 20 or 25 per cent bone mass (or BMC or bone density) loss, e.g. up to 40 per cent bone (or BMC or bone density) loss.

It is preferred to co-administer an estrogen.

This is conveniently administered at the same time as the progestin and if desired also on a non-cyclical basis.

Representative estrogens of the invention include conjugated estrogens, e.g. conjugated equine estrogens, oestriol and esters thereof, e.g. oestriol 16,17-bis-hemi-succinate, ethinyl oestradiol and derivatives thereof, e.g. quinestrol, mestranol and moxestrol. Preferred is oestriol.

Particularly preferred estrogens include oestradiol (estradiol), preferably 17-beta-estradiol and derivatives thereof such as ethers, e.g. promestriene, and esters thereof, e.g. estradiol hemi-succinate, and more conveniently fatty acid esters such as estradiol benzoate, estradiol 17-beta-cypionate, estradiol 3,17-beta-cypionate, and estradiol 17-beta-undecylate, estradiol 17-beta-valerate, estradiol 17-beta-propionate, estradiol 3,17-beta-dipropionate, and estradiol 17-heptonate.

The especially preferred estrogen is (17-beta-estradiol).

The progestins and estrogens may be administered by any conventional route, e.g. nasally, parenterally, particularly in the form of implants, or in particular enterally, preferably orally, particularly in the form of tablets or capsules, or transdermally or percutaneously, e.g. in the form of a patch or gels.

Such compositions may be formulated in conventional manner to bein conventional forms, for example, capsules, tablets, implants, suppositories, dispersible powders, syrups, elixirs, suspensions or solutions, appropriate for the mode of administation chosen. For example transdermal patches containing steroids may be made in analogous manner to that described in USP 4,291,014, 4,397,459, 4,321,252 or 4,438,139.

Such compositions conveniently contain more than 1% by weight of the progestin and/or estrogen. Suitable pharmaceutical diluents or carriers include, for example, water, alcohols, natural or hardened oils and waxes, calcium and sodium carbonates, calcium phosphate, kaolin, talc and lactose as well as suitable preserving agents, such as ethyl-p-hydroxybenzoate, suspending agents such as methyl cellulose, tragacanth and sodium alginate, wetting agents such as lecithin, polyoxyethylene stearate and polyoxyethylene sorbitan mono-oleate, granulating and disintegrating agents such as starch and alginic acid, binding agents such as starch, gelatin and acacia, and lubricating agents such as magnesium stearate, stearic acid and talc, in order to provide an elegant and palatable pharmaceutical preparation.

Compositions in tablet form may be coated by conventional techniques to delay disintegration of the tablet and absorption of the active ingredient in the gastrointestinal tract and thereby provide sustained action over a long period.

Suitable daily dosages of progestins and the estrogen (if present) to be given in the continuous treatment of the method of the invention will be readily apparent since dosages used may be those daily doses contemplated in fixed combinations on a continuous or cyclical basis for the prevention of osteoporosis in menopausal women.

The exact dosage will of course vary depending on e.g. the particular progestin and/or estrogen chosen, the mode of administation and the nature and severity of the condition being treated.

However, in general, for oral administration daily dosages from about 10 microgram to about 10 mg of the progestin or estrogen are suitable.

Alternatively transdermal patches may contain dosages of progestin and/or estrogen suitable for administration over one to seven days. Implants may contain dosages of progestin and/or estrogen suitable for administration over several months.

A particularly preferred oral composition contains a) NETA and b) 17-beta-oestradiol and optionally c) oestriol, especially 1 mg of NETA and 2 mg of 17-beta-oestradiol, and optionally 1 mg oestriol.

Preferably the active agents are all micronized.

Such a composition is commercially available under the brand KLIOGEST (Trademark of Novo, Denmark).

The invention furthermore provides a pack containing a progestin as defined above together with instructions for use in the method of the invention. Such packs may be in the form of a calender pack, comprising e.g. a number of compositions of the invention arranged in a particular order.

It is also preferred to co-administer a calcium preparation, e.g. calcium gluconolactate.

Suitable calcium preparations are in general known, e.g. as described in GB 2,192,131 A.

Typically about 300 mg to 1000 mg, e.g. 300 to 800 mg, calcium ions are administered daily.

## Claims

1. The use of a progestin unsubstituted in the 19 position in the manufacture of a medicament for the treatment of established osteoporosis on a non-cyclical basis.

2. The use of a progestin unsubstituted in the 19 position in the manufacture of a medicament for increasing bone mass in the treatmetn of established osteoporosis on a non-cyclical basis.

3. A use according to claim 1 or 2 wherein the progestin is to be administered over more than 25 days per month.

4. A use according to claim 1 or 2 wherein the progestin is chosen from the following: allylestrenol, demegestone, ethynodiol diacetate, gestonorone caproate, dl- and levo-noresterel, lynestrenol, methyl estrenolone morethynodrel, norgestrienone, and quinestradiol or its acetate.

5. A use according to any claims 1,2 or 3 wherein the progestin is a northesterone or a derivative thereof.

6. A use according to claim 5 wherein the progestin is norethisterone acetate.

7. A use according to claim 6 wherein the progestin is administered at 1 mg per day.

8. A use according to any preceding claim wherein the progestin is to be administered to women over 60 years of age.

9. A use according to any preceding claim wherein the progestin is administered orally.

10. A use according to any one of claims 1 to 8 wherein the progestin is administered transdermally.

11. A use according to any preceding claim wherein the progestin is administered concomitantly with an estrogen.

12. A use according to claim 11 wherein the estrogen is administered on a non-cylical basis.

13. A use according to claims 11 or 12 wherein the estrogen is administered in the prgestin medicament.

14. A use according to claim 11,12 or 13 whereint the estrogen is a conjugated estrogen.

15. A use according to claim 11,12 or 13 wherein the estrogen is oestriol or an ester thereof.

16. A use according to claim 11,12 or 13 wherein the estrogen is 17-beta oestradiol.

17. A use according to claim 7 wherein the medicament contains 1 mg norethisterone acetate and 2 mg 17-beta oestradiol.

18. A use according to claim 17 wherein the medicament contains at least for some days of the month additionally 1 mg oestriol.

19. A use or method substantially as hereinbefore described with reference to the example.

20. A method of treating established osteoporosis on a non-cyclical basis which comprises admnistering a progestin as defined in any preceding claim to a subject in need of such treatment.

21. A pack containing a progestin as defined in any preceding claim together with instructions for use in a method as defined in claim 20.

FIG. 1

EP 0 337 938 A2

FIG. 2

EP 0 337 938 A2

# FIG. 3